(19) Europäisches Patentamt
European Patent Office
Office européen des brevets

(11) Veröffentlichungsnummer: **0 003 992**
**A2**

# (12) EUROPÄISCHE PATENTANMELDUNG

(21) Anmeldenummer: 79100495.5

(22) Anmeldetag: 20.02.79

(51) Int. Cl.[2]: **C 07 D 499/70**
C 07 D 499/00, A 61 K 31/43
//C07D413/12, C07D405/12,
C07D417/12

(30) Priorität: 08.03.78 DE 2810083

(43) Veröffentlichungstag der Anmeldung:
19.09.79 Patentblatt 79/19

(84) Benannte Vertragsstaaten:
BE CH DE FR GB IT NL

(71) Anmelder: Bayer Aktiengesellschaft
Zentralbereich Patente,Marken und Lizenzen Bayerwerk
D-5090 Leverkusen 1(DE)

(72) Erfinder: Preiss, Michael, Dr.
Egenstrasse 21
D-5600 Wuppertal-1(DE)

(72) Erfinder: König, Hans-Bodo, Dr.
Herberts-Katernberg 10
D-5600 Wuppertal-1(DE)

(72) Erfinder: Metzger, Karl-Georg, Dr.
Pahlkestrasse 15
D-5600 Wuppertal-1(DE)

(72) Erfinder: Schmidt, Gunter, Dr.
Pahlkestrasse 63
D-5600 Wuppertal-1(DE)

## (54) Beta-lactam Verbindungen, Verfahren zu ihrer Herstellung und ihre Verwendung als Arzneimittel.

(57) ß-Lactamverbindungen der Formel

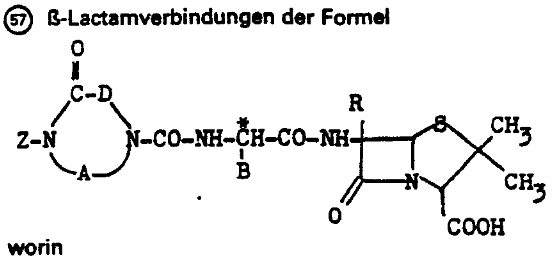

(I)

worin
R Wasserstoff oder Alkoxy, B einen heterocyclischen Rest, A Äthylen, Trimethylen oder o-Phenylen, D eine Carbonylgruppe oder eine direkte Bindung und Z Wasserstoff oder einen Substituenten bedeuten, zeigen neben guter Verträglichkeit und Löslichkeit eine breite antibakterielle Wirkung sowie die Eigenschaft, das Wachstum und die Futterverwertung bei Tieren zu verbessern.

EP 0 003 992 A2

Croydon Printing Company Ltd.

BEZEICHNUNG GEÄNDERT
siehe Titelseite

BAYER AKTIENGESELLSCHAFT
Zentralbereich
Patente, Marken und Lizenzen

5090 Leverkusen, Bayerwerk
Jo/bc

## ß-Lactam-Verbindungen

Die Erfindung betrifft ß-Lactam-Verbindungen, Verfahren zu ihrer Herstellung sowie ihre Verwendung als Arzneimittel, insbesondere als antimikrobielle Mittel und als Mittel zur Förderung des Wachstums und zur Verbesserung der Futterverwertung bei Tieren.

Die erfindungsgemäßen ß-Lactam-Verbindungen entsprechen in Form der freien Säure der Formel

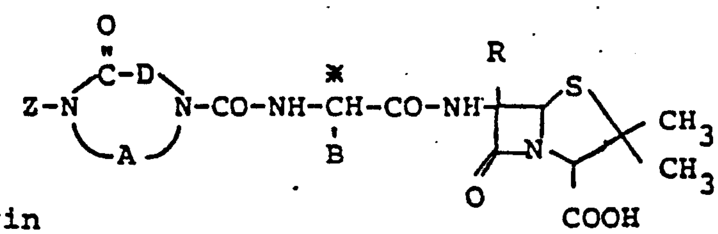

(I)

worin

R Wasserstoff oder Alkoxy,

B gegebenenfalls substituiertes Furyl, Pyrrolyl, Pyrazolyl, Imidazolyl, Triazolyl, Tetrazolyl, Oxazolyl, Isoxazolyl, Oxdiazolyl, Thiazolyl, 2-Imino-$\Delta^4$-thiazolin-

4-yl, Isothiazolyl, Thiadiazolyl, Oxtriazolyl, Thiatriazolyl, Sydnonyl, Pyridyl, Pyrazinyl, Pyridazinyl, Pyrimidinyl, Triazinyl, Tetrazinyl oder Pyronyl,

A Äthylen, Trimethylen oder o-Phenylen,

D eine Carbonylgruppe oder eine direkte Bindung und

Z Wasserstoff, gegebenenfalls substituiertes Alkyl oder Alkenyl, gegebenenfalls substituiertes Cycloalkyl, Cycloalkenyl oder Cycloalkadienyl, gegebenenfalls substituiertes Aralkyl, gegebenenfalls substituiertes Aryl, gegebenenfalls substituiertes Heterocyclyl, oder Acyl bedeuten.

Geeignete Gruppen R sind insbesondere niedere Alkoxygruppen, vor allem $C_1$-$C_4$-Alkoxy.

Als Substituenten der Reste B kommen Halogen wie Fluor, Chlor und Brom, Alkyl mit 1 bis 6 C-Atomen, Cyano, Nitro, Hydroxy, Amino Sulfo und Methylsulfonyl in Frage; die Reste B können auch partiell hydriert sein. Die Zahl der möglichen Substituenten ist vorzugsweise 0 bis 2. Bevorzugte Substituenten sind Fluor, Chlor, Methyl und Äthyl, Cyano, Hydroxy und Amin

Gegebenenfalls substituiertes Alkyl Z bedeutet geradkettiges oder verzweigtes Alkyl mit vorzugsweise 1 bis 6, insbesondere 1 bis 4 Kohlenstoffatomen, das vorzugsweise durch 1 bis 2 gleiche oder verschiedene Substituenten $R^3$ substituiert sein kann. Gegebenenfalls substituiertes Alkenyl Z ist geradkettiges oder verzweigtes Alkenyl mit vorzugsweise 2 bis 6, insbesondere 2 bis 4 Kohlenstoffatomen,

das durch 1 bis 2 gleiche oder verschiedene Substituenten $R^3$ substituiert sein kann. Gegebenenfalls substituiertes Cycloalkyl, Cycloalkenyl und Cycloalkadienyl Z ist mono-, bi- oder tricyclisch und enthält vorzugsweise 3 bis 10, insbesondere 3, 5 oder 6 Kohlenstoffatome und kann durch 1 oder 2 gleiche oder verschiedene Substituenten $R^3$ substituiert sein. Vorzugsweise sind die Alkyl-, Alkenyl-, Cycloalkyl-, Cycloalkenyl- und Cycloalkadienyl-Gruppen unsubstituiert oder enthalten einen Substituenten $R^3$.

$R^3$ bedeutet vorzugsweise Halogen, insbesondere Fluor, Chlor und Brom, Amino, $C_1$-$C_4$-Alkylamino, vorzugsweise Methylamino oder Äthylamino, Di-$C_1$-$C_4$-Alkylamino, vorzugsweise Dimethylamino oder Diäthylamino, Pyrrolidyl, Piperidyl, Formylamino, $C_1$-$C_4$-Alkylcarbonylamino, vorzugsweise Acetylamino, N-$C_1$-$C_4$-Alkyl-formylamino, vorzugsweise N-Methyl-formylamino oder N-Äthyl-formylamino, N-$C_1$-$C_4$-Alkyl-$C_1$-$C_4$-alkylcarbonylamino, vorzugsweise N-Äthyl-acetylamino, Di-$C_1$-$C_4$-Alkylmethylimino, $C_1$-$C_4$-Alkylsulfonylamino, vorzugsweise Methylsulfonylamino oder Äthylsulfonylamino, N-$C_1$-$C_4$-Alkyl-$C_1$-$C_4$-alkylsulfonylamino, vorzugsweise N-Methyl-methylsulfonylamino, Hydroxysulfonylamino, N-$C_1$-$C_4$-Alkyl-hydroxysulfonylamino, vorzugsweise N-Methyl-hydroxysulfonylamino oder N-Äthyl-hydroxysulfonylamino, Amidino, Di-$C_1$-$C_4$-Alkylamino-methylimino, insbesondere Dimethylamino-methylimino, Pyrrolidinylmethylimino, Guanido, Nitro, Azido, Hydroxy, $C_1$-$C_4$-Alkoxy, vorzugsweise Methoxy oder Äthoxy, Formyloxy, $C_1$-$C_4$-Alkylcarbonyloxy, vorzugsweise Acetoxy, Äthylcarbonyloxy oder tert.-Butylcarbonyloxy, $C_1$-$C_4$-Alkoxycarbonyloxy, vorzugsweise Methoxycarbonyloxy, Äthoxycarbonyloxy

oder tert.-Butylbutoxycarbonyloxy, Aminocarbonyloxy, $C_1$-$C_4$-Alkylaminocarbonyloxy, vorzugsweise Methylaminocarbonyloxy oder Äthylaminocarbonyloxy, Di-$C_1$-$C_4$-Alkylaminocarbonyloxy, vorzugsweise Dimethylaminocarbonyloxy oder Diäthylaminocarbonyloxy, Piperidinylcarbonyloxy, Aminosulfonyloxy, $C_1$-$C_4$-Alkylaminosulfonyloxy, vorzugsweise Methylaminosulfonyloxy oder Äthylaminosulfonyloxy, Di-$C_1$-$C_4$-Alkylaminosulfonyloxy, vorzugsweise Methylaminosulfonyloxy oder Diäthylaminosulfonyloxy, Carboxy, Aminocarbonyl, Di-$C_1$-$C_4$-Alkylaminocarbonyl, insbesondere Dimethylaminocarbonyl und Diäthylaminocarbonyl, Formyl, Hydroxysulfonyloxy, Mercapto, $C_1$-$C_4$-Alkylmercapto, vorzugsweise Methylmercapto, Trifluormethylmercapto, Äthylmercapto oder Isopropylmercapto, $C_1$-$C_4$-Alkylsulfinyl, vorzugsweise Methylsulfinyl oder Äthylsulfinyl, Sulfo, $C_1$-$C_4$-Alkylsulfonyl, vorzugsweise Methylsulfonyl, Trifluormethylsulfonyl oder Äthylsulfonyl, Aminosulfonyl, $C_1$-$C_4$-Alkylaminosulfonyl, vorzugsweise Methylaminosulfonyl oder Äthylaminosulfonyl, Di-$C_1$-$C_4$-Alkylaminosulfonyl, vorzugsweise Dimethylaminosulfonyl oder Diäthylaminosulfonyl, Piperidinylsulfonyl, Thiosulfato, Phenoxy und - im Falle der cyclischen Reste - Alkyl mit 1 bis 6 Kohlenstoffatomen, vorzugsweise Methyl.

Gegebenenfalls substituiertes Aralkyl Z enthält 6 oder 10, insbesondere 6,Kohlenstoffatome im Arylrest und vorzugsweise 1 bis 4, insbesondere 1 oder 2 Kohlenstoffatome im Alkylteil, wobei der Alkylteil geradkettig oder verzweigt sein kann und der Aralkylrest 1 bis 3 Substituenten $R^3$

tragen kann. Vorzugsweise ist der Aralkylrest unsubstituiert oder enthält einen Substituenten $R^3$.

Gegebenenfalls substituiertes Aryl Z ist insbesondere Aryl mit 6 bis 10 Kohlenstoffatomen, das durch 1 bis 3 Substituenten $R^3$ substituiert sein kann. Insbesondere ist Aryl gegebenenfalls durch 1 Substituenten $R^3$ substituiertes Phenyl oder Naphthyl.

Als gegebenenfalls substituiertes Heterocyclyl Z stehen heteroparaffinische, heteroaromatische und heteroolefinische 5- bis 7-gliedrige, vorzugsweise 5- oder 6-gliedrige Ringe mit vorzugsweise 1 bis 3, insbesondere 1 oder 2, gleichen oder verschiedenen Heteroatomen. Als Heteroatome kommen Sauerstoff, Schwefel oder Stickstoff in Frage. Als Beispiele seien gegebenenfalls substituiertes Thienyl, Furyl, Oxazolyl, Isoxazolyl, Thiazolyl, Isothiazolyl, Pyrrolyl, Imidazolyl, Pyrazolyl, Oxadiazolyl, Thiadiazolyl, Triazolyl, Oxtriazolyl, Thiatriazolyl, Tetrazolyl, Pyridyl, Pyrazinyl, Pyrimidinyl, Tetrahydrofuranyl, Dioxanyl, Pyrrolidinyl, Piperidinyl, Morpholinyl, Pyronyl-2 und Pyronyl-4 in Frage. Heterocyclyl Z kann durch 1 oder mehrere, vorzugsweise 1 bis 3, insbesondere 1 oder 2 gleiche oder verschiedene Substituenten $R^4$ substituiert sein. Ganz besonders bevorzugt ist Heterocyclyl Z unsubstituiert oder enthält einen Substituenten $R^4$.

Für den Fall, daß $R^4$ an ein Kohlenstoffatom gebunden ist, bedeutet $R^4$ vorzugsweise $C_1$-$C_4$-Alkyl, vorzugsweise Methyl, Äthyl oder Isopropyl, Trifluormethyl, Halogen, vorzugsweise Fluor, Chlor oder Brom, Amino, $C_1$-$C_4$-Alkylamino, vorzugsweise Methylamino oder Äthylamino, Di-$C_1$-$C_4$-

Alkylamino, vorzugsweise Dimethylamino oder Diäthylamino, Formylamino, Acetylamino, Methoxycarbonylamino, Äthoxycarbonylamino, Methylsulfonylamino, Hydroxy, Methoxy, Äthoxy, Methylmercapto, Äthylmercapto, Methylsulfonyl, Methylsulfinyl, Carboxy, Sulfo, Formyl, $C_1$-$C_4$-Alkylcarbonyl, vorzugsweise Acetyl, $C_1$-$C_4$-Alkoxycarbonyl, vorzugsweise Methoxycarbonyl oder Äthoxycarbonyl und Cyano.

Für den Fall, daß $R^4$ an ein Stickstoff gebunden ist, bedeutet $R^4$ $C_1$-$C_4$-Alkyl, vorzugsweise Methyl, Äthyl, Propyl oder Isopropyl, Cyano, Formyl, $C_1$-$C_4$-Alkoxycarbonyl, vorzugsweise Methoxycarbonyl, Äthoxycarbonyl, Isopropoxycarbonyl, tert.-Butoxycarbonyl, Aminocarbonyl, $C_1$-$C_4$-Alkylaminocarbonyl, vorzugsweise Methylaminocarbonyl, Äthylaminocarbonyl oder Isopropylaminocarbonyl und $C_1$-$C_4$-Alkylcarbonyl, vorzugsweise Acetyl, Äthylcarbonyl oder Isopropylcarbonyl.

Acylreste Z sind insbesondere gegebenenfalls substituiertes Alkylcarbonyl, Arylcarbonyl, Alkylsulfonyl, Arylsulfonyl, Alkoxycarbonyl, Aminocarbonyl und Aminosulfonyl. Gegebenenfalls substituiertes Alkylcarbonyl ist insbesondere $C_1$-$C_4$-Alkylcarbonyl. Gegebenenfalls substituiertes Arylcarbonyl ist insbesondere gegebenenfalls durch einen oder mehrere, vorzugsweise 1 bis 3, insbesondere 1 oder 2, gleiche oder verschiedene Substituenten $R^3$ substituiertes Phenylcarbonyl. Gegebenenfalls substituiertes Alkylsulfonyl ist insbesondere $C_1$-$C_4$-Alkylsulfonyl. Gegebenenfalls substituiertes Arylsulfonyl ist insbesondere gegebenenfalls durch ein oder mehrere, vorzugsweise 1 bis 3, insbesondere 1 oder 2, gleiche oder verschiedene Sub-

stituenten $R^3$ substituiertes Phenylsulfonyl. Gegebenenfalls substituiertes Alkoxycarbonyl ist vorzugsweise $C_1$-$C_4$-Alkoxycarbonyl. Gegebenenfalls substituiertes Aminocarbonyl und Aminosulfonyl ist gegebenenfalls durch $C_1$-$C_4$-Alkyl mono- oder disubstituiertes Aminocarbonyl und Aminosulfonyl.

Die Carboxylgruppe der Formel (I) kann als freie Carboxylgruppe, als pharmazeutisch verwendbare Estergruppe, in Form eines Salzes oder durch eine geeignete Schutzgruppe geschützt vorliegen.

Die Verbindungen der Formel (I) können bezüglich des Chiralitätszentrums $\overset{*}{C}$ in den beiden möglichen R- und S-Konfigurationen sowie als Gemische der daraus resultierenden Diastereomeren vorliegen. Bevorzugt sind die erfindungsgemäßen Verbindungen, in welchen $\overset{*}{C}$ in der D-Konfiguration (R-Konfiguration) vorliegt.

Alle Kristallformen und Hydratformen der erfindungsgemäßen Verbindungen der allgemeinen Formel (I) und ihrer Salze sind in gleicher Weise antibakteriell wirksam.

Die neuen ß-Lactam-Verbindungen der Formel (I) werden erhalten, indem man Verbindungen, die in Form der freien Säure der Formel (II)

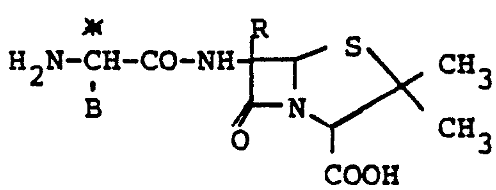

(II)

entsprechen, worin

R, B und $\overset{*}{C}$ die oben angegebene Bedeutung haben,

mit Verbindungen der Formel (III)

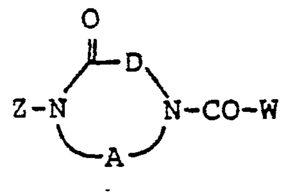

(III)

worin

Z, D und A die oben angegebene Bedeutung haben und

W für eine nukleofuge Abgangsgruppe steht,

in Gegenwart eines Lösungsmittels und gegebenenfalls eines säurebindenden Mittels bei Temperaturen von etwa -20°C bis etwa +50°C umsetzt und die erhaltenen ß-Lactam-Verbindungen gegebenenfalls in ihre pharmazeutisch verwendbare Salze oder Ester überführt oder aus den erhaltenen Salzen gewünschtenfalls die freien Säuren herstellt.

Unter nukleofugen Abgangsgruppen in der Definition von W sind alle üblicherweise in der organischen Chemie verwendeten nukleofugen Gruppen und vor allem solche zu verstehen, welche in Angewandte Chemie, <u>81</u> (1969), 543 beschrieben sind. Vorzugsweise verwendete nukleofuge Abgangsgruppen sind Halogenatome und Azid-Gruppen.

Die erfindungsgemäßen Verbindungen der Formel (I), worin R für Alkoxy steht, sind auch durch Alkoxylierung der entsprechenden Verbindungen, worin R Wasserstoff bedeutet, herstellbar, wobei es vorteilhaft ist, bei der Alkoxylierung solche Verbindungen zu verwenden, in denen die Carboxylgruppe durch eine Schutzgruppe geschützt ist.

Die Verbindungen der Formel (I) sind weiterhin dadurch erhältlich, daß man Verbindungen der Formel (IV)

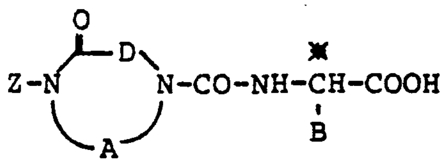

(IV)

worin

B, D, A und Z die oben angegebene Bedeutung haben und

die Carboxylgruppe nach den aus der Peptid-Chemie bekannten Methoden aktiviert ist, mit Verbindungen, die in Form der freien Säure der Formel (V)

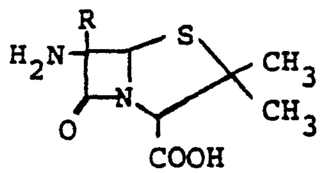

(V)

entsprechen, umsetzt.

Die erfindungsgemäßen Verbindungen zeigen neben guter Verträglichkeit und Löslichkeit eine breite antibakterielle Wirkung, d.h. Wirkung gegenüber mehreren Bakterienfamilien im gram-negativen Bereich und gegenüber ß-Lactamasebildnern. Aufgrund ihrer starken antibakteriellen Eigenschaften und aufgrund ihrer Fähigkeit, das Wachstum und die Futterverwertung bei Tieren zu verbessern, stellen die erfindungsgemäßen Verbindungen somit eine Bereicherung der Technik dar.

Gegenüber ähnlichen, vorbekannten Verbindungen, beispielsweise solchen aus DT-OS 2 104 579, 2 104 580, 2 152 967, 2 152 968, 2 258 973, 2 407 715, 2 528 078 und 2 528 079, sowie aus US-PS 3 974 141, 3 983 105, 3 972 869 und 4 031 229 zeichnen sich die erfindungsgemäßen Verbindungen durch höhere Wirksamkeit und/oder bessere Verträglichkeit aus.

Bevorzugte Verbindungen innerhalb der Formel (I) sind solche, die in Form der freien Säure der allgemeinen Formel (VI)

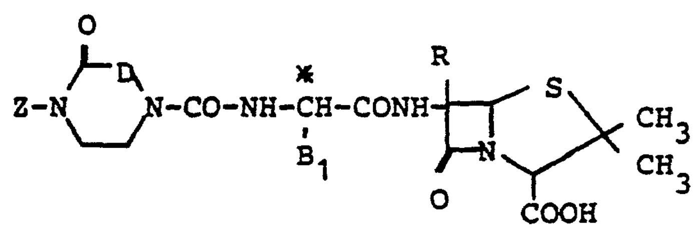

(VI)

entsprechen, worin

Z und D die oben angegebene Bedeutung haben,

R Wasserstoff oder Methoxy und

$B_1$ Furyl, Pyrrolyl, Pyrazolyl, Imidazolyl, Thiazolyl, Isothiazolyl, Oxazolyl, Isoxazolyl, Thiadiazolyl, Oxdiazolyl, Iminothiazolyl, Pyridyl, Pyrimidinyl, Pyridazinyl, Pyrazinyl, Sydnonyl, Tetrazolyl oder 2-Imino-$\Delta^4$-thiazolin-4-yl bedeuten und

das Chiralitätszentrum C* in der D-Konfiguration (R-Konfiguration) vorliegt. Besonders bevorzugt sind die Natriumsalze dieser Verbindungen.

Ganz besonders bevorzugte Verbindungen der Formel (VI) sind solche, in denen Z Wasserstoff, Methyl, Äthyl, Propyl, Cyclopropyl, Methylsulfonyl, Äthylsulfonyl, Phenylsulfonyl, Methylaminosulfonyl oder Thiazolyl ist.

Die als Ausgangsstoffe verwendeten Verbindungen der allgemeinen Formel (II) sind bereits bekannt oder nach bekannten Methoden erhältlich. Alle Kristallformen, Hydratformen und Salze der Verbindungen der allgemeinen Formel (II) sind als Ausgangsmaterialien für das erfindungsgemäße Verfahren geeignet.

Als Beispiele seien genannt:
6-(α-Aminofuryl)-acetamido-penicillansäure, 6-[α-amino-(2-imino-$\Delta^4$-thiazolidin-4-yl)-acetamidopenicillansäure, 6-[α-Amino-(5-methylisoxazol-3-yl)-acetamido-penicillansäure und 6-(α-Aminopyrid-3-yl)-acetamidopenicillansäure. Als Salze der Verbindungen der Formel (II) können vorzugsweise Salze mit Basen eingesetzt werden, welche als für die Salzbildung mit Verbindungen der Formel (I) geeignet aufgeführt werden. Besonders bevorzugt sind Dinatriumsalze.

Die als Ausgangsstoffe verwendeten Verbindungen der allgemeinen Formel (III) sind nach bekannten Methoden erhältlich (DE-OS 25 28 078, 21 52 967).

Als Beispiele für die erfindungsgemäßen Ausgangsverbindungen der allgemeinen Formel (III) seien genannt: 1-Chlorcarbonyl-2-oxo-3-methylsulfonylimidazolin, 1-Azidocarbonyl-2-oxo-3-methylsulfonylimidazolin, 1-Chlorcarbonyl-2-oxoimidazolin, 1-Chlorcarbonyl-2-oxo-3-cyclopropyl-imidazolin und 1-Chlorcarbonyl-2,3-dioxo-4-äthyl-piperazin.

Diejenigen Verbindungen der allgemeinen Formel (III), in denen W Azid ist, werden in üblicher Weise, z.B. aus den entsprechenden Halogenverbindungen durch Umsetzung mit Alkaliaziden erhalten.

Als Verdünnungsmittel kommen beim erfindundungsgemäßen Verfahren Wasser sowie alle inerten organischen Lösungsmittel, vorzugsweise solche, welche mit Wasser mischbar sind, in Frage. Hierzu gehören vor allem niedere Dialkylketone, z.B. Aceton, Methyläthylketon, cyclische Äther, z.B. Tetrahydrofuran und Dioxan; Nitrile, z.B. Acetonitril; niedere Dialkylformamide, z.B. Dimethylformamid; niedere Alkanole, z.B. Aethanol und Isopropanol sowie Dimethylsulfoxid. Diese Lösungsmittel können auch in Mischungen untereinander sowie in beliebigen Mischungen einzelner oder mehrerer dieser Lösungsmittel mit Wasser verwendet werden. Das erfindungsgemäße Verfahren kann also durchgeführt werden in Gegenwart von: (a) ausschließlich Wasser, (b) ausschließlich einem oder mehreren organischen Lösungsmitteln oder (c) Wasser und einem oder mehreren organischen Lösungsmitteln. Ist wegen des Vorhandenseins von Wasser eine pH-Messung während der erfindungsgemäßen Reaktion möglich, wird der pH der Reaktionsmischung durch Zusatz von Basen oder durch Verwendung von Puffergemischen vorzugsweise zwischen 6,5 bis 7,5 gehalten. Das erfindungsgemäße Verfahren läßt sich aber auch sehr gut in einem anderen pH-Bereich, beispielsweise zwischen 4,5 und 9,0 oder bei pH 2,0 bis 4,5, durchführen. Ferner ist es möglich, die Reaktion in mit Wasser nicht mischbaren Lösungsmitteln, z.B. halogenierten Kohlenwasserstoffen, wie Chloroform oder Methylenchlorid, unter Zusatz von organischen Basen, vorzugsweise Niederalkylaminen, z.B. Triäthylamin, Diäthylamin oder cyclischen Basen, z.B. N-Äthylpiperidin durchzuführen. Weiterhin läßt sich die Reaktion in einer Mischung aus Wasser und einem mit Wasser nicht mischbaren Lösungsmittel, wie z.B. Niederalkyläthern, wie Diäthyläther, halogenierten Kohlenwasserstoffen, wie Chloroform und Methylenchlorid; Schwefelkohlenstoff; Isobutylmethylketon; Estern wie Essigsäureäthylester; aromatischen Kohlenwasserstoffen wie Benzol, ausführen, wobei es zweckmäßig ist, kräftig zu rühren und den pH-Wert durch Basenzusatz oder Verwendung von üblichen Pufferlösungen, z.B. Phosphat-, Acetat- oder Citratpuffer, zwischen 4,5 und 9,0 oder z.B. 2,0 und 4,5 zu halten. Man kann die Reaktion aber auch in Wasser allein in Abwesenheit von organischen Lösungsmitteln in Gegenwart einer organischen oder anorganischen Base oder unter Zusatz von üblichen Pufferstoffen durchführen.

Als Säurebindemittel können alle in der Chemie der Antibiotica üblicherweise verwendeten Säurebinder verwendet werden. Hierzu gehören anorganische Basen und organische Basen, welche z.B. durch sterische Hinderung schwer acylierbar sind. Als Beispiele für anorganische Basen seien Natrium- und Kaliumhydroxid genannt. Als organische Basen kommen praktisch alle nicht oder schwer acylierbaren offenkettigen oder cyclischen Amine und auch heteroaromatische Basen in Frage. Als Basen seien beispielhaft tertiäre Amine, vorzugsweise Niederalkylamine, z.B. Triäthylamin und/ oder cyclische Basen, z.B. Pyridin sowie als schwer acylierbares sekundäres Amin Dicyclohexylamin genannt.

Beim erfindungsgemäßen Verfahren ist der Zusatz einer Base nur dann erforderlich, wenn während der Reaktion saure Verbindungen entstehen, z.B. im Falle, daß W für Halogen oder Azid steht.

Die Reaktionstemperaturen können in einem größeren Bereich variiert werden. Im allgemeinen arbeitet man zwischen etwa -20°C und etwa +50°C, vorzugsweise zwischen 0 und +20°C. Wie bei den meisten chemischen Reaktionen können jedoch prinzipiell auch höhere oder niedrigere Temperaturen verwendet werden.

Die Umsetzung kann bei Normaldruck, aber auch bei vermindertem oder erhöhtem Druck ausgeführt werden. Im allgemeinen arbeitet man bei Normaldruck.

Bei der Durchführung der erfindungsgemäßen Verfahren können die Anteile der Reaktionspartner der Formel (II) und (III) weiten Grenzen variiert werden, ohne daß das Ergebnis nachteilig beeinflußt wird. Die Ausgangsstoffe können z.B. in äquimolekularen Mengen miteinander zur Reaktion gebracht werden. Es kann jedoch zweckmäßig sein, einen der beiden Reaktionspartner im Überschuß zu verwenden, um so die Reinigung oder Reindarstellung des gewünschten Penicillins zu erleichtern und die Ausbeute zu erhöhen.

Beispielsweise kann man die Reaktionspartner der allgemeinen Formel (II) mit einem Überschuß von 0,1 bis 0,3 Moläquivalenten einsetzen und dadurch eine geringere Zersetzung der Reaktionspartner der allgemeinen Formel (III) in einem wasserhaltigen Lösungsmittelgemisch erreichen. Der Überschuß der Reaktionspartner der allgemeinen Formel (II) läßt sich wegen der guten Löslichkeit in wäßrigen Mineralsäuren beim Aufarbeiten des Reaktionsgemisches leicht entfernen.

Andererseits kann man aber auch mit Vorteil die Reaktionspartner der allgemeinen Formel (III) mit einem Überschuß von beispielsweise 0,1 bis 1,0 Moläquivalenten einsetzen. Dadurch werden die Reaktionspartner der allgemeinen Formel (II) besser ausgenützt und die als Nebenreaktion in wasserhaltigen Lösungsmitteln ablaufende Zersetzung der Reaktionsteilnehmer der allgemeinen Formel (III) kompensiert. Da die im Überschuß zugesetzten Verbindungen der allgemeinen Formel (III) sich in Wasser rasch in neutrale stickstoffhaltige Heterocyclen umwandeln, die sich leicht entfernen lassen, wird die Reinheit der Antibiotica hierdurch kaum beeinträchtigt.

Die Menge der gegebenenfalls verwendeten Basen ist z.B.

durch die gewünschte Einhaltung eines bestimmten pH-Wertes festgelegt. Wo eine pH-Messung und Einstellung nicht erfolgt oder wegen des Fehlens von ausreichenden Mengen Wasser im Verdünnungsmittel nicht möglich ist oder nicht sinnvoll ist, werden vorzugsweise 2 Moläquivalente Base zugesetzt.

Die Aufarbeitung der Reaktionsansätze zur Herstellung der erfindungsgemäßen Verbindungen und ihrer Salze erfolgt durchweg in der bei diesen Körpern allgemein bekannten Art und Weise. Auch die Isolierung und Reinigung der erfindungsgemäßen Verbindungen sowie die Freisetzung der freien Säuren aus Salzen oder die Umwandlung der freien Säuren in Salze werden nach allgemein üblichen Methoden der organischen Chemie, welche jedem Fachmann geläufig sind, vorgenommen.

Alternativ sind die Verbindungen der Formel (I), bei denen R Alkoxy bedeutet, auch durch Alkoxylierung der entsprechenden Wasserstoffderivate (R=H) erhältlich, wobei die Carboxylgruppe durch eine geeignete Schutzgruppe, z.B. eine leicht entfernbare esterbildende Gruppe oder eine Acetoxymethylgruppe geschützt ist oder als Salz, vorzugsweise als Alkali- oder Erdalkalimetallsalz vorliegt.

Bei diesem Verfahren setzt man eine ß-Lactam-Verbindung der Formel (I), in der R Wasserstoff bedeutet, mit 2 bis 10 Äquivalenten pro Äquivalent ß-Lactam-Verbindung einer Base in Anwesenheit eines Überschusses eines Alkanols

in einem inerten organischen Lösungsmittel um, fügt zwischen etwa 1 bis etwa 8 Äquivalente eines N-Halogenierungsmittels zu und isoliert die Verbindung der Formel (I) in der R Alkoxy bedeutet, gegebenenfalls nach vorheriger Abspaltung der Säureschutzgruppe, Überführung in ein Salz oder einen pharmazeutisch verwendbaren Ester.

Bei diesem erfindungsgemäßen Verfahren werden als N-Halogenierungsmittel, vorzugsweise positives Chlor übertragende Verbindungen, wie t-Butylhypochlorit oder Chloracetamid, verwendet.

Als Basen eignen sich komplexe und einfache, vorzugsweise jedoch einfache Alkali- und Erdalkalihydride, metallorganische Verbindungen sowie Grignard-Verbindungen. Als Beispiele seien genannt: Lithiumhydrid, Natriumhydrid, Butyl-Lithium, Phenyl-Lithium, Alkyl-Magnesiumbromid, beispielsweise Methyl-Magnesiumbromid, oder andere bekannte Säurebindemittel wie Alkali- und Erdalkali-alkoholate oder -carbonate, Alkali- und Erdalkali-bicarbonate oder -oxide wie beispielsweise Natriumcarbonat oder andere Säurebindemittel wie Borax oder offenkettige oder cyclische organische Basen wie Trialkyl- oder Aralkylamine oder cyclische Amidine wie 2,3,4,6,7,8-Hexahydro-pyrrolo/1,2-a/pyrimidin (DBN) oder 2,3,4,6,7,8,9,10-Octahydro-pyrimido/1,2-a/azepin (DBU).

Als Lösungsmittel eignen sich beispielsweise offenkettige oder cyclische Äther, aliphatische und aromatische Kohlenwasserstoffe oder Halogenkohlenwasserstoffe oder die Alkohole R'OH. Besonders geeignet ist Tetrahydrofuran.

Pharmazeutisch verwendbare Salze der Verbindungen der Formel (I) sind Salze dieser Verbindungen mit anorganischen und organischen Basen an der sauren Carboxylgruppe bzw. den sauren Carboxyl- und Sulfonsäuregruppen. Als Basen können hierzu alle in der pharmazeutischen Chemie, insbesondere in der Chemie der Antibiotika, üblicherweise verwendeten Basen eingesetzt werden. Als anorganische Basen seien beispielhaft genannt: Alkali- und Erdalkalihydroxide, Alkali- und Erdalkalicarbonate und Alkalihydrogencarbonate, wie Natrium- und Kaliumhydroxid, Calcium- und Magnesiumhydroxid, Natrium- und Kaliumcarbonat, Calciumcarbonat, Natrium- und Kaliumhydrogencarbonat; Aluminiumhydroxid und Ammoniumhydroxid. Als organische Amine können primäre, sekundäre und tertiäre aliphatische Amine sowie heterocyclische Amine eingesetzt werden. Beispielhaft seien genannt: Di- und Trialkylamine, z.B. Diäthylamin, Triäthylamin, Tri-ß-hydroxyäthylamin, Procain, Dibenzylamin, N,N'-Dibenzyläthylendiamin, N-Benzyl-ß-phenyl-äthylamin, N-Methyl- und N-Äthylmorpholin, 1-Ephenamin, Dehydroabietylamin, N,N'-Bis-dehydroabietyläthylendiamin, N-$C_1$-$C_4$-Alkylpiperidin. Auch sogenannte basische Aminosäuren wie Lysin oder Arginin können vorteilhaft als Basen Verwendung finden. Besonders bevorzugte Salze sind Dinatriumsalze.

Die erfindungsgemäßen Wirkstoffe weisen bei geringer Toxizität eine starke und breite antimikrobielle Wirksamkeit auf. Diese Eigenschaften ermöglichen ihre Verwendung als chemotherapeutische Wirkstoffe in der Medizin sowie als Stoffe zur Konservierung von anorganischen und

organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Wirkstoffe sind gegen ein sehr breites Spektrum von Mikroorganismen wirksam. Mit ihrer Hilfe können gramnegative und grampositive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Wirkstoffe gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Micrococcaceae, wie Staphylokokken, z.B. Staphylococcus aureus, Staph.epidermidis, Staph.aerogenes und Gaffkya tetragena (Staph. = Staphylococcus); Lactobacteriaceae, wie Streptokokken, z.B. Streptococcus pyogenes, $\alpha$- bzw. ß-hämolysierende Streptokokken, nicht ($\gamma$-)-hämolysierende Streptokokken, Str.viridans, Str.faecalis (Enterokokken), Str.agalactiae, Str.lactis, Str.equi, Str.anaerobis und Diplococcus pneumoniae (Pneumokokken) (str. = Streptococcus); Neisseriaceae, wie Neisserien, z.B. Neisseria gonorrhoeae (Gonokokken), N.meningitidis (Meningokokken), N.catarrhalis und N.flava (N. = Neisseria); Corynebacteriaceae, wie Corynebakterien, z.B. Corynebacterium diphtheriae, C.pyogenes, C.diphtheroides, C.acnes, C.parvum, C.bovis, C.renale, C.ovis, C.murisepticum.

Mycobacteriaceae, wie Erreger von Mykobakteriosen, z.B. Mycobacterium tuberculosis, M.bovis, M.avium, sogenannte atypische Mykobakterien der Runyon-Gruppen I, II, III und IV, M.leprae (M. = Mycobacterium); Enterobacteriaceae, wie Escherichiae-Bakterien der Coli-Gruppe: Escherichia-Bakterien, z.B. Escherichia coli, Enterobacter-Bakterien, z.B. E.aerogenes, E.cloacae, Klebsiella-Bakterien, z.B. K.pneumoniae, K.pneumoniae, K.ozaenae, Erwiniae, z.B. Erwinia spec., Serratia, z.B. Serratia marcescens (E. = Enterobacter) (K. = Klebsiella), Proteae-Bakterien der Proteus-Gruppe: Proteus, z.B. Proteus vulgaris, Pr.morganii, Pr.rettgeri, Pr.mirabilis, Providencia, z.B. Providencia sp. (Pr. = Proteus), Salmonelleae: Salmonella-Bakterien, z.B. Salmonella paratyphi A und B, S.typhi, S.enteritidis, S.cholerae suis, S.typhimurium (S. = Salmonella, Shigella-Bakterien, z.B. Shigella dysenteriae, Sh. ambigua, Sh.flexneri, Sh.boydii, Sh.sonnei (Sh. = Shigella);

Pseudomonadaceae, wie Pseudomonas-Bakterien, z.B. Pseudomonas aeruginosa, Ps.pseudomallei (Ps. = Pseudomonas), Aeromonas-Bakterien, z.B. Aeromonas liquefaciens, A. hydrophila (A. = Aeromonas);

Parvobacteriaceae, wie Pasteurella-Bakterien, z.B. Pasteurella multocida, Past.pestis (Yersinia), Past.pseudotuberculosis, Haemophilus-Bakterien, z.B. Haemophilus influenzae, H.ducreyi, H.suis, H.canis, H.aegypitcus (H. = Haemophilus) , Bordetella-Bakterien, z.B. B.bronchiseptica (B. = Bordetella).

Bacteroidaceae, wie Bacteroides-Bakterien, z.B. Bacteroides fragilis, B.serpens (B. = Bacteroides), Fusiforme-Bakterien, z.B. Fusobacterium fusiforme, Sphaerophorus-Bakterien, z.B. Sphaerophorus necrophorus, Sph.necroticus, Sph.pyrogenes (Sph. = Sphaerophorus);
Bacillaceae, wie aerobe Sporenbildner, z.B. Bacillus anthracis (B.subtilis, B.cereus) B. = Bacillus), Anaerobe Sporenbildner-Chlostridien, z.B. Clostridium perfringens, Cl.septicium, Cl.oedematien, Cl.histolyticum, Cl.tetani, Cl.botulinum (Cl. = Clostridium).

Die obige Aufzählung von Erregern ist lediglich beispielhaft und keineswegs beschränkend aufzufassen.

Als Krankheiten, die durch die erfindungsgemäßen Wirkstoffe verhindert, gebessert und/oder geheilt werden können, seien beispielsweise genannt:

Erkrankungen der Atmungswege und des Rachenraumes;

Otitis; Pharyngitis; Pneumonie; Peritonitis; Pyelonephritis; Cystitis; Endocarditis; Systeminfektionen; Bronchitis; Arthritis; lokale Infektionen.

Zur vorliegenden Erfindung gehören pharmazeutische Zubereitungen, die neben nichttoxischen,inerten pharmazeutisch

geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen

enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyäthylenglykole oder Gemische der unter (a) bis (i) aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einleitungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyäthylenglykole, Fette, z.B.

Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyäthylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z.B. Wasser, Aethylalkohol, Isopropylalkohol, Aethylcarbonat, Aethylacetat, Benzylalkohol, Benzylbenzoat, Propylenglykol, 1,3-Butylenglykol, Dimethylformamid, Oele, insbesondere Baumwollsaatoel, Erdnußoel, Maiskeimoel, Olivenoel, Ricinusoel und Sesamoel, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z. B. Wasser, Aethylalkohol, Propylenglykol, Suspendiermittel, z.B. äthoxylierte Isostearylalkohole, Polyoxyäthylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, in der Human- und Veterinärmedizin zur Verhütung, Besserung und/oder Heilung der oben aufgeführten Erkrankungen.

Die Wirkstoffe oder die pharmazeutischen Zubereitungen können lokal, oral, parenteral, intraperitoneal und/oder rectal, vorzugsweise oral oder parenteral wie intravenös oder intramuskulär appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 5 bis etwa 1000, vorzugsweise 20 bis 200 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe, vorzugsweise in Mengen von etwa 1 bis etwa 250, insbesondere 10 bis 100 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der

Wirkstoffe kann durch jeden Fachmann aufgrund seines Fachwissens leicht erfolgen.

Im Falle der Anwendung als Futterzusatzmittel können die neuen Verbindungen in den üblichen Konzentrationen und Zubereitungen zusammen mit dem Futter bzw. mit Futterzubereitungen oder mit dem Trinkwasser gegeben werden. Dadurch kann eine Infektion durch gramnegative oder grampositive Bakterien verhindert, gebessert und/oder geheilt werden und ebenso eine Förderung des Wachstums und eine Verbesserung der Verwertung des Futters erreicht werden.

Die neuen Penicilline und Cephalosporine zeichnen sich durch starke antibakterielle Wirkungen, die in vivo und in vitro geprüft wurden, aus.

Die erfindungsgemäßen Penicilline und Cephalosporine können zum Zwecke der Erweiterung des Wirkungsspektrums und um eine Wirkungssteigerung speziell bei ß-lactamasebildenden Bakterien zu erzielen mit anderen antimikrobiellen Wirkstoffen, z.B. mit Penicillinen, die besonders penicillinasefest sind, kombiniert werden. Eine solche Kombination wäre z.B. die mit Oxacillin oder Dicloxacillin.

Die erfindungsgemäßen Penicilline können zum Zwecke der Erweiterung des Wirkungsspektrums und um eine Wirkungssteigerung zu erreichen auch mit Aminoglycosid-Antibiotika, wie Gentamicin, Kanamicin, Sisomicin, Amikacin, Tobramicin, Lactamaseinhibitoren wie Clavulansäure und Clavulansäurederivaten kombiniert werden.

Die Wirksamkeit der erfindungsgemäßen ß-Lactamantibiotika kann durch die folgenden in vitro- und in vivo-Versuche beispielhaft demonstriert werden:

1. In vitro-Versuche

Die Beispiele 1 und 2, welche als typische Vertreter der erfindungsgemäßen Verbindungen betrachtet werden können, wurden mit Müller-Hinton-Nährbrühe unter Zusatz von 0,1 % Glucose auf einen Gehalt von 100 $\mu$g/ml verdünnt. In der Nährlösung befanden sich jeweils $1 \times 10^5$ bis $2 \times 10^5$ Bakterien pro Milliliter. Die Röhrchen mit diesem Ansatz wurden jeweils 24 Stunden bebrütet und anschließend wurde der Trübungsgrad bestimmt. Trübungsfreiheit gibt Wirkung an. Bei der Dosierung von 100 $\mu$g/ml waren die folgenden Bakterienkulturen trübungsfrei (sp. = species):

Klebsiella pneumoniae; Enterobacter aerogenes sp.; Providencia; Serratia mercescens; E.coli BE; Salmonella sp.; Shigella sp.; Proteus, indolnegativ und indolpositiv; Pasteurella pseudotuberculosis; Brucella sp.; Haemophilus influenzae; Bordetella bonchiseptica; Staphylococcus aureus 133; Neisseria catarrhalis sp.; Diplococcus pneumoniae sp.; Streptococcus pyogenes W.; Enterococcus sp.; Lactobacillus sp.; Corynebacterium diphteriae gravis; Corynebacterium pyogenes M; Clostridium tetani; Pseudomonas aeruginosa sp.

Beispiel 1

Natrium-6-{α-[(2-oxo-imidazolidin-1-yl)-carbonylamino]-furyl-2-acetamido}-penicillanat

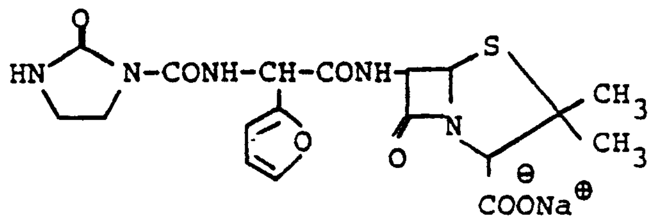

Eine Lösung von 2,4 g 6-(α-Amino-furylacetamido)-penicillansäure (US-PS 3 120 514) in 50 ml 80 %igem wäßrigem Tetrahydrofuran (THF) wird auf 5°C gekühlt und mit 1 N Natronlauge auf pH 8 gebracht. 1,4 g 1-Chlorcarbonyl-2-oxo-imidazol werden portionsweise zugefügt, währenddessen der pH mit 0,5 N Natronlauge auf 7,5 gehalten wird. Wenn der pH-Wert konstant ist, werden 50 ml Wasser zugegeben und das THF im Vakuum abdestilliert. Die wäßrige Lösung wird mit Essigsäureäthylester extrahiert, auf 5°C gekühlt, mit 150 ml Essigsäureäthylester überschichtet und mit 1 N Salzsäure angesäuert, ausgeschüttelt, die organische Phase abgetrennt, über $MgSO_4$ getrocknet und eingeengt. Der Rückstand wird in 35 ml Wasser suspendiert, mit 0,5 N Natronlauge gelöst und diese Lösung lyophilisiert. Man erhält 2,5 g Natriumsalz vom Zersetzungspunkt 200 bis 210°C. IR (Paraffinöl): 3200, 1770, 1705, 1600 $cm^{-1}$.

Beispiel 2

Natrium-6-{α-[(2-oxo-3-methylsulfonyl-imidazolidin-1-yl)-carbonylamino]-furyl-2-acetamido}-penicillanat

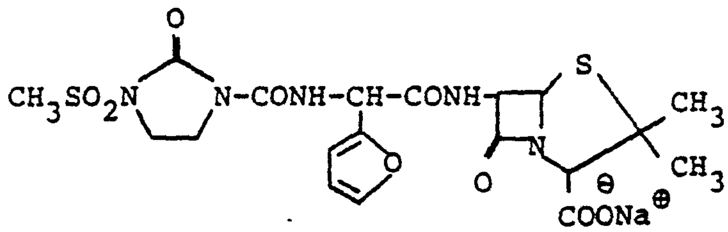

2,4 g 6-(α-Amino-furylacetamido)-penicillansäure und 2,3 g 1-Chlorcarbonyl-2-oxo-3-methylsulfonyl-imidazol werden wie in Beispiel 1 umgesetzt und aufgearbeitet. Man erhält 3,2 g vom Zersetzungspunkt 195 bis 204°C. IR (KBr): 3420, 1750, 1660, 1590, 1525, 1380, 1165 $cm^{-1}$.

Wird von D,L-6-(α-Amino-furfurylacetamido)-penicillansäure ausgegangen, so erhält man die Penicilline von Beispiel 1 und 2 als Diastereomerengemische, die beispielsweise mittels präparativer Hochdruckflüssigkeitschromatographie in die α-D- und α-L-Formen getrennt werden können.

Beispiel 3.1

2-(2-Oxo-3-methylsulfonyl-imidazolidin-1-yl-carbonylamino)-(5-methyl-isoxazol-3-yl)-essigsäure

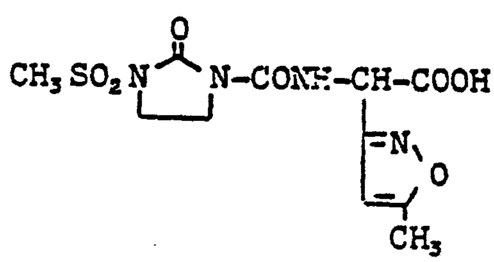

Zu einer Suspension von 8,5 g 2-(5-Methylisoxazol-3-yl)-glycin in 150 ml Wasser wird bis zur Auflösung 1 N Natronlauge gegeben und danach der pH auf 7,6 gebracht. Dann werden 12,3 g 1-Chlorcarbonyl-2-oxo-3-methylsulfonyl-imidazolidin portionsweise zugefügt, wobei der pH durch

Zutropfen von 1 N Natronlauge bei 7,5 - 7,7 gehalten wird. Es wird abgesaugt, angesäuert und mit Essigester extrahiert. Die Essigesterphasen werden über Natriumsulfat getrocknet und eingeengt. Es resultieren 11,0 g vom Zers.p. 168°C, die am Hochvakuum bei 100°C getrocknet werden.
IR (KBr): 3326, 1737, 1536, 1396, 1168 $cm^{-1}$.

Beispiel 3.2

Natrium-6- {α-[(2-oxo-3-methylsuflonyl-imidazolidin-1-yl)-carbonylamino]-(5-methyl-isoxazol-3-yl)-acetamido} -penicillanat

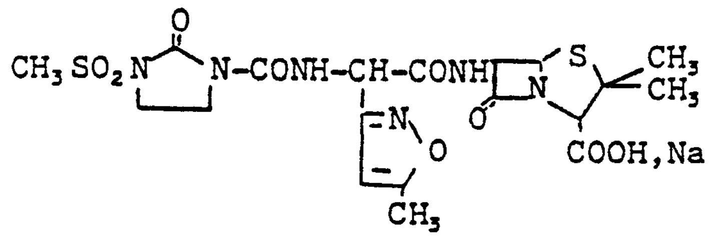

Zu einer Suspension von 3,8 g 2-(3-Methylsulfonyl-2-oxo-imidazolidin-1-yl)-carbonylamino-(5-methyl-isoxyzol-3-yl)-essigsäure in 100 ml absolutem Aceton werden 1,1 g Triäthylamin gegeben. Das Gemisch wird 30 Minuten gerührt, dann auf -40°C gekühlt, mit 0,1 g 3-Dimethylaminopropanol-1 und 0,5 g Chlorameisensäure-äthylester versetzt, 20 Minuten bei -20°C gerührt und dann auf -50°C abgekühlt. Dazu werden auf einmal eine -10°C kalte Lösung von 3,6 g 6-Aminopenicillansäure in 15 ml Wasser und 10 ml Aceton mit 2 N Natronlauge bei 0°C gelöst) gegeben. Man erwärmt auf Raumtemperatur, gibt 35 ml Wasser zu, zieht das Aceton ab, gibt Wasser bis zu einem Volumen von 120 ml hinzu, filtriert, säuert auf pH 2 an und saugt das Produkt ab, das in Wasser suspendiert, mit 2 N Natronlauge zu einer ca. 10-proz. Lösung gelöst und gefriergetrocknet wird. Man erhält 3,6 g vom Zers.-Punkt 186-205°C.
IR (KBr): 3440, 1770, 1730, 1675, 1605, 1395, 1167 $cm^{-1}$.

<u>Beispiel 4.1</u>

2-(2,3-Dioxo-4-äthyl-piperazin-1-yl-carbonylamino)-furyl-2-essigsäure

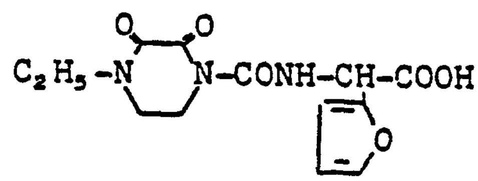

13,8 g 2-Furylglycin in 250 ml Wasser werden mit 20,0 g 1-Chlorcarbonyl-2,3-dioxo-4-äthyl-piperazin wie in Beispiel 3.1 umgesetzt. Man erhält 13,2 g vom Zers.p. 202 - 207°C.
IR (KBr): 3279, 1745, 1713, 1659, 1491, 1391, 1287 1180, 756 $cm^{-1}$.

<u>Beispiel 4.2</u>

Natrium-6-{α-/(2,3-dioxo-4-äthyl-piperazin-1-yl)-carbonylamino/- furyl-2-acetamido} -penicillanat

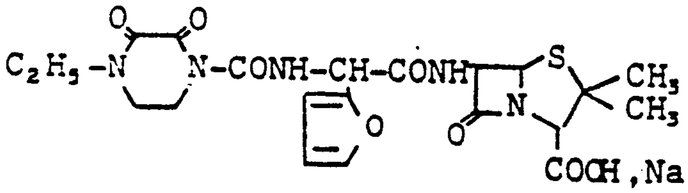

3,4 g 2-(2,3-Dioxo-4-äthyl-piperazin-1-yl-carbonylamino)-furylessigsäure werden mit 3,6 g 6-Aminopenicillansäure wie in Beispiel 3.2 umgesetzt. Man erhält 2,8 g vom Zers.p. 215-220°C.
IR (KBr): 3410, 1769, 1607, 1459, 1339, 1310 $cm^{-1}$.

Beispiel 5.1

2-(2-Oxo-3-methylsulfonyl-imidazolidin-1-yl-carbonylamino)-(5-methylfuryl)-essigsäure

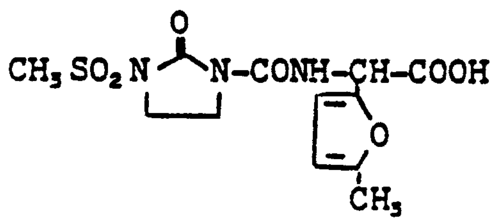

13,7 g 2-(Methylfuryl)-glycin /hergestellt wie 2-Furylglycin/ werden in 250 ml Wasser mit 1-Chlorcarbonyl-2-oxo-3-methylsulfonyl-imidazolidin wie in Beispiel 3.1 umgestzt. Das Produkt fällt beim Ansäuern aus und wird abgesaugt. Man erhält 12,8 g vom Zers.p. 184-189°C.
IR (KBr): 3987, 1745, 1661, 1548, 1478, 1392, 1344, 1164 $cm^{-1}$.

Beispiel 5.2

Natrium-6-{α-[(2-oxo-3-methylsulfonyl-imidazolidin-1-yl)-carbonylamino]-(5-methyl-furyl-2-)acetamido}-penicillanat

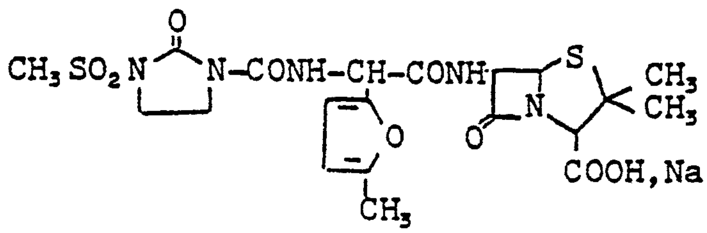

3,8 g 2-(2-oxo-3-methylsulfonyl-imidazolidin-1-yl-carbonylamino)-(5-methlfuryl-2-)essigsäure werden mit 2,5 g 6-Aminopenicillansäure wie in Beispeil 3.2 umgesetzt. Man erhält 3,8 g vom Zers.p. 193-195°C.
IR (KBr): 3430, 1766, 1731, 1677. 1611, 1520, 1478, 1394, 1171 $cm^{-1}$.

Entsprechend den beiden Beispielen können weitere wertvolle Wirkstoffe hergestellt werden, die in der folgenden Tabelle angegeben sind.

Tabelle

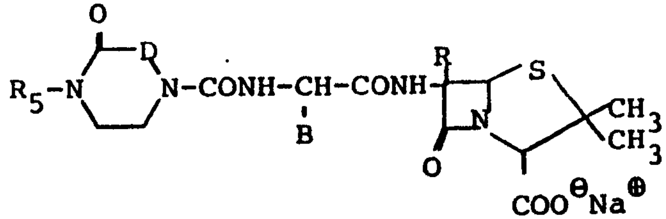

| R | B | $R_5$ | D |
|---|---|---|---|
| H | Furyl-2 | H | - |
| H | Furyl-2 | Methylsulfonyl | - |
| H | Furyl-2 | Cyclopropyl | - |
| H | Furyl-2 | Methylsulfonyl | C=O |
| $OCH_3$ | Furyl-2 | H | - |
| H | Furyl-3 | Methylaminosulfonyl | - |
| H | Furyl-3 | Cyclopropyl | - |
| $OCH_3$ | Furyl-3 | Cyclopropyl | - |
| H | Furyl-3 | Methylsulfonyl | C=O |
| H | Pyrrolyl-2 | H | - |
| $OCH_3$ | Pyrrolyl-2 | H | - |
| H | Pyrrolyl-2 | Methylsulfonyl | C=O |
| H | Pyrrolyl-2 | Cyclopropyl | - |
| H | Pyridinyl-3 | Methylaminosulfonyl | - |
| $OCH_3$ | Pyridinyl-3 | H | - |

**Tabelle** (Fortsetzung)

| R | B | $R_5$ | D |
|---|---|---|---|
| H | Pyridinyl-3 | Cyclopropyl | - |
| H | Pyridinyl-3 | Cyclopropyl | C=O |
| H | Pyridinyl-3 | Methylsulfonyl | - |
| H | Imidazolyl-2 | H | - |
| H | Imidazolyl-2 | Methylsulfonyl | - |
| $OCH_3$ | Imidazolyl-2 | Methylsulfonyl | - |
| H | Imidazolyl-2 | Methylsulfonyl | C=O |
| H | Imidazolyl-2 | Methylaminosulfonyl | - |
| H | 1-Methylimidazolyl-2 | H | - |
| H | 1-Methylimidazolyl-2 | Methylsulfonyl | - |
| $OCH_3$ | Isothiazolyl-5 | H | - |
| H | Isothiazolyl-5 | Methylsulfonyl | - |
| H | Isothiazolyl-5 | Methylaminosulfonyl | - |
| H | Thiazolyl-4 | H | - |
| H | Thiazolyl-4 | Methylsulfonyl | - |
| $OCH_3$ | Thiazolyl-4 | Methylaminosulfonyl | - |
| H | Thiazolyl-4 | H | C=O |
| H | 5-Methylisoxazolyl-3 | H | - |

Tabelle (Fortsetzung)

| R | B | $R_5$ | D |
|---|---|---|---|
| H | 5-Methylisoxazolyl-3 | Cyclopropyl | - |
| $OCH_3$ | 5-Methylisoxazolyl-3 | Cyclopropyl | - |
| H | 2-Imino-$\Delta^4$-thiazolinyl-4 | Methylaminosulfonyl | - |
| $OCH_3$ | 2-Imino-$\Delta^4$-thiazolinyl-4 | Cyclopropyl | - |
| H | 2-Imino-$\Delta^4$-thiazolinyl-4 | Cyclopropyl | - |

Beispiel 6 :

a) 2-(2-Oxo-imidazolidin-1-yl-carbonylamino)-2-(2-benzyl-oxycarbonylamino-thiazol-4-yl)-essigsäure

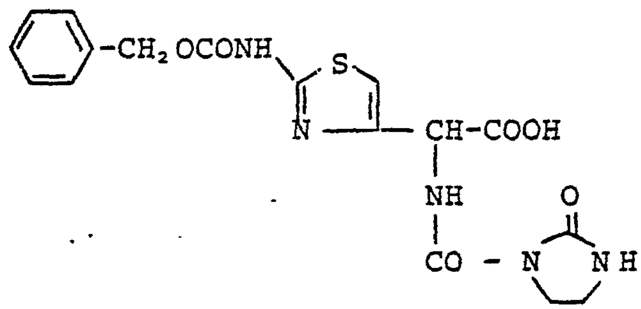

13,5 g (0,0394 Mol) α-Amino-α-(2-benzyloxycarbonylamino-thiazol-4-yl)-essigsäure•dihydrat werden in 100 ml Wasser und 100 ml THF suspendiert und mit 2N Natronlauge bei einem pH-Wert von 7.8 klar gelöst. 6,4 g (0,0433 Mol) 1-Chlorcarbonyl-2-oxo-imidazolidin werden in fester Form portionsweise innerhalb von 30 Min. bei 0°C und bei einem pH-Wert von 7.5 bis 7.8 hinzugegeben. Anschließend läßt man die Lösung noch 2 Stunden bei Raumtemperatur rühren, wobei der pH-Wert durch Zugabe von 2N Natronlauge bei 7.5 konstant gehalten wird. Danach wird THF abdestilliert, die verbleibende Lösung mit 200 ml Wasser verdünnt und mit Essigester extrahiert. Die wäßrige Phase wird mit 2N HCl auf pH 2 angesäuert und mit Essigester mehrmals extrahiert. Die vereinigten Essigesterphasen werden mit kochsalzhaltigem Wasser gewaschen, über Natriumsulfat getrocknet und das Lösungsmittel abdestilliert.

Ausbeute 9,5 g (58%).

b) Natrium-6-{2-[(2-oxo-imidazolidin-1-yl)-carbonylamino]-2-(2-benzyloxycarbonylamino-thiazol-4-yl)-acetamido}-penicillinat

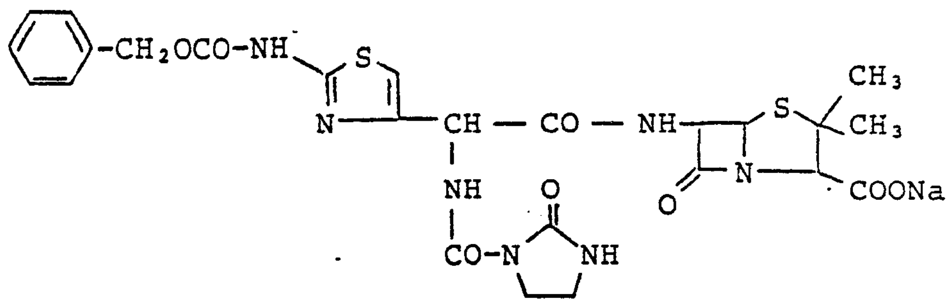

9,4 g (0,0224 Mol) 2-(2-Oxo-imidazolidin-1-yl-carbonylamino)-2-(2-benzyloxycarbonylamino-thiazol-4-yl)-essigsäure werden in 60 ml THF und 60 ml DMF mit 32 ml (0,0228 Mol) Triäthylamin unter Zusatz von 4 Tropfen N-Methylmorpholin versetzt. Bei -20°C setzt man 2,94 ml (0,0224 Mol) Chlorameisensäureisobutylester unter Rühren hinzu. Nach 40 Min. bei -15 bis -10°C gibt man eine vorgekühlte Natriumsalzlösung von 6-Aminopenicillansäure (6-APS) - hergestellt aus 4,85 g (0,0224 Mol) 6-APS - hinzu. Man läßt 2 Stunden bei einem pH-Wert von 7.1 auf 10 bis 15°C erwärmen. Danach wird die Lösung mit 150 ml Wasser versetzt, ein pH-Wert von 7.6 eingestellt und zweimal mit Essigester gewaschen. Die wäßrige Phase wird bei 0°C mit 2N HCl angesäuert und mit Essigester extrahiert. Die Essigesterphasen werden mit NaCl-Lösung gewaschen, getrocknet und i.Vak. eingeengt. Aus dem Rückstand wird mit 1M Lösung von Natrium-2-äthylhexanoat in methanolischem Äther das Natriumsalz gebildet.

Ausbeute 9,5 g (63%).

c) Natrium-6-{2-[(2-oxo-imidazolidin-1-yl)-carbonylamino]-2-(2-amino-thiazol-4-yl)-acetamido}-penicillinat

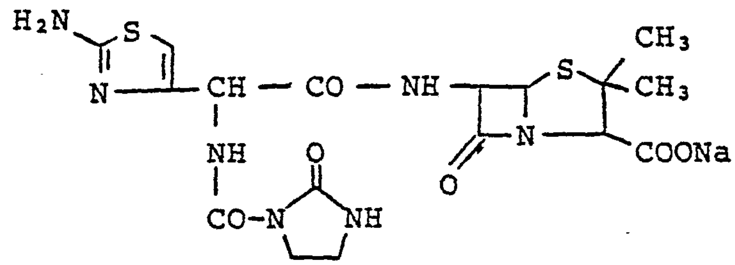

9,5 g (0,0141 Mol) Natrium-6-{2-[(2-oxo-imidazolidin-1-yl)-carbonylamino]-2-(2-benzyloxycarbonylamino-thiazol-4-yl)-acetamido}-penicillinat werden in 200 ml Wasser gelöst und in einer vorhydrierten, wäßrigen Lösung (300 ml) über 40 g Palladium-Mohr hydriert. Nach einer Stunde wird der Katalysator abgetrennt und die Lösung gefriergetrocknet.

Ausbeute 3,4 g ( 43% )

$^1$H-NMR ($CD_3OD$; 100 MHz):

$\delta$ = 1.55-1.60 (2α-$CH_3$, 2β-$CH_3$),
3.37-3.56 (m, 2H, Imidazolidinon),
3.79-4.0 (m, 2H, Imidazolidinon),
4.2 (s, 3-H), 5.44-5.53 (d, 5-H, 6-H),
6.52 ppm (s, 1-Thiazol-H).

Beispiel 7 :

a) 2-(2-Oxo-3-methylsulfonyl-imidazolidin-1-yl-carbonylamino)-2-(2-benzyloxycarbonylamino-thiazol-4-yl)-essigsäure

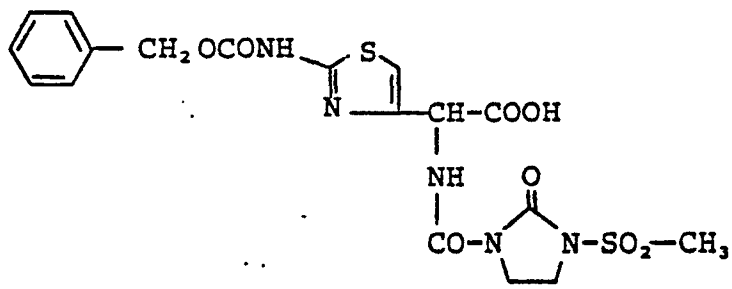

Das Thiazolglycin-Derivat wird analog Beispiel 6a aus 15 g (0,0437 Mol) α-Amino-α-(2-benzyloxycarbonylamino-thiazol-4-yl)-essigsäure · dihydrat in THF/Wasser (1:1) und 10,9 g (0,0481 Mol) 1-Chlorcarbonyl-2-oxo-3-methylsulfonyl-imidazolidin in einem pH-Bereich von 7.5 bis 8.0 hergestellt.

Ausbeute 14,1 g ( 65% ).

b) Natrium-6-{2-[(2-oxo-3-methylsulfonyl-imidazolidin-1-yl)-carboxylamino]—2-(2-benzyloxycarbonylamino-thiazol-4-yl)-acetamido}-penicillinat

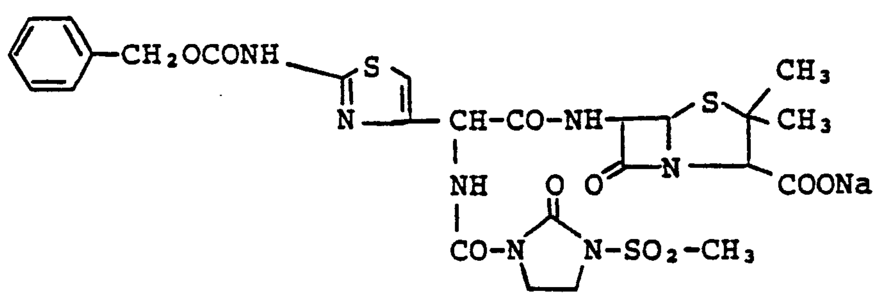

Das Carbobenzoxy-geschützte Penicillinderivat wird analog Beispiel 6b aus 14,1 g (0,0284 Mol) 2-(2-Oxo-3-methylsulfony imidazolidin-1-yl-carbonylamino)-2-(2-benzyloxycarbonylaminc thiazol-4-yl)-essigsäure hergestellt, die zur Bildung des gemischten Anhydrids mit 3,98 ml (0,028 Mol) Triäthylamin unter Zusatz von 5 Tropfen N-Methylmorpholin und 3,74 ml (0,0284 Mol) Chlorameisensäureisobutylester behandelt wird. Das gemischte Anhydrid wird danach mit 6,14 g (0,0284 Mol) 6-APS umgesetzt.

Ausbeute 16,1 g ( 77% ).

c) Natrium-6-{2-[(2-oxo-3-methylsulfonyl-imidazolidin-1-yl)-carbonylamino]-2-(2-amino-thiazol-4-yl)-acetamido}-penic.

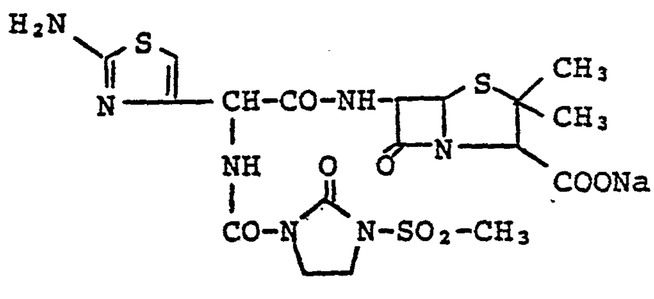

14 g (0,019 Mol) Natrium-6-{2-[(2-oxo-3-methylsulfonyl-imidazolidin-1-yl)-carbonylamino]-2-(2-benzyloxycarbonylar thiazol-4-yl)-acetamido}-penicillinat werden analog Beispiel 6c in Wasser über 40 g Palladium-Mohr hydriert.

Ausbeute 8 g ( 70% ).

$^1$H-NMR ($CD_3OD$, 100 MHz):
$\delta$ = 1.52-1.60 (2 α-$CH_3$, 2 ß-$CH_3$),
3.32 (s, 3H, Methylsulfonyl),
3.9 (s, 4H, Imidazolidin-H),
4.17 (s, 3-H), 5.48 (s, 5-H, 6-H),
6.57 ppm (s, 1-Thiazol-H).

Patentansprüche

1. ß-Lactam-Verbindungen, die in Form der freien Säure der Formel

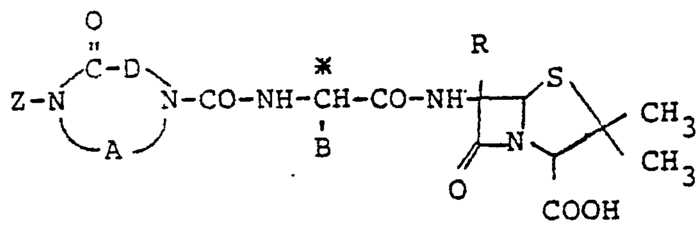

(I)

entsprechen, worin

R Wasserstoff oder Alkoxy,

B gegebenenfalls substituiertes Furyl, Pyrrolyl, Pyrazolyl, Imidazolyl, Triazolyl, Tetrazolyl, Oxyzolyl, Isoxazolyl, Oxdiazolyl, Thiazolyl, 2-Imino-$\Delta^4$-thiazolin-4-yl, Isothiazolyl, Thiadiazolyl, Oxtriazolyl, Thiatriazolyl, Sydnonyl, Pyridyl, Pyrazinyl, Pyridazinyl, Pyrimidinyl, Triazinyl, Tetrazinyl oder Pyronyl,

A Äthylen, Trimethylen oder o-Phenylen,

D eine Carbonylgruppe oder eine direkte Bindung und

Z Wasserstoff, gegebenenfalls substituiertes Alkyl oder Alkenyl, gegebenenfalls substituiertes Cycloalkyl, Cycloalkenyl oder Cycloalkadienyl, gegebenenfalls substituiertes Aralkyl, gegebenenfalls substituiertes Aryl, gegebenenfalls substituiertes Heterocyclyl oder Acyl bedeuten.

2. ß-Lactam-Verbindungen nach Anspruch 1, die in Form der freien Säure der allgemeinen Formel (VI)

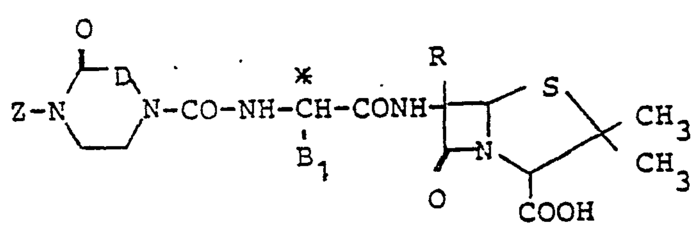

(VI)

entsprechen, worin

Z und D die in Anspruch 1 angegebene Bedeutung haben,

R Wasserstoff oder Methoxy und

$B_1$ Furyl, Pyrrolyl, Pyrazolyl, Imidazolyl, Thiazolyl, Isothiazolyl, Oxazolyl, Isoxazolyl, Thiadiazolyl, Oxdiazolyl, Iminothiazolyl, Pyridyl, Pyrimidinyl, Pyridazinyl, Pyrazinyl, Sydnonyl, Tetrazolyl oder 2-Imino-$\Delta^4$-thiazolin-4-yl bedeuten und

das Chiralitätszentrum $\overset{*}{C}$ in der D-Konfiguration (R-Konfiguration) vorliegt.

3. ß-Lactamverbindungen nach Anspruch 1 als Natriumsalze.

4. ß-Lactamverbindungen nach Anspruch 3, worin Z Wasserstoff, Methyl, Äthyl, Propyl, Cyclopropyl, Methylsulfonyl, Äthylsulfonyl, Phenylsulfonyl, Methylaminosulfonyl oder Thiazolyl ist.

5. Natrium-6-{α-[(2-oxo-imidazolidin-1-yl)-carbonylamino]-furyl -2-acetamido} -penicillanat.

6. Verfahren zur Herstellung von ß-Lactam-Verbindungen gemäß Anspruch 1, dadurch gekennzeichnet, daß man Verbindungen, die in Form der freien Säure der Formel

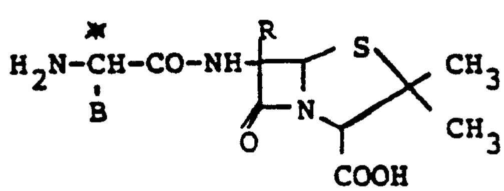

entsprechen, worin B und R die in Anspruch 1 genannte Bedeutung besitzen, mit Verbindungen der Formel

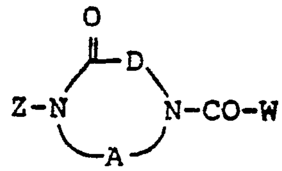

worin D, A und Z die in Anspruch 1 genannte Bedeutung besitzen und W für eine nukleofuge Abgangsgruppe steht, in Gegenwart eines Lösungsmittels und gegebenenfalls eines säurebindenden Mittels umsetzt.

7. Verfahren zur Herstellung von Verbindungen gemäß Anspruch 1, worin R Alkoxy bedeutet, dadurch gekennzeichnet, daß man Verbindungen gemäß Anspruch 1, worin R Wasserstoff bedeutet, alkoxyliert.

8. Verfahren zur Herstellung von ß-Lactamverbindungen gemäß Anspruch 1, dadurch gekennzeichnet, daß man Verbindungen der Formel

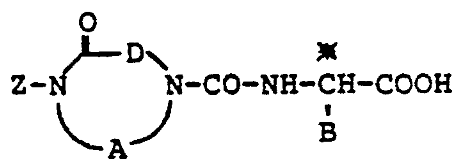

worin B, D, A und Z die in Anspruch 1 angegebene Bedeutung haben und die Carboxylgruppen nach den aus der Peptid-Chemie bekannten Methoden aktiviert ist, mit Verbindungen der Formel

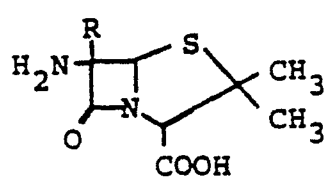

worin R Wasserstoff oder Alkoxy bedeutet, umsetzt.

9. Verwendung der ß-Lactamverbindungen gemäß Anspruch 1 als antibakterielle Mittel.

10. Arzneimittel, gekennzeichnet durch einen Gehalt an Penicillinen gemäß Anspruch 1.